# EUROPEAN PATENT APPLICATION

(11) **EP 4 220 074 A1**
(43) Date of publication of application: **02.08.2023**
(21) Application number: 22153870.5
(22) Date of filing: 28.01.2022
(51) Int. Cl.: G01B 11/24, A61B 5/00, G01N 21/57, G06T 7/00

(54) **DETERMINING A PARAMETER MAP FOR A REGION OF A SUBJECT'S BODY**

(71) Applicant: Koninklijke Philips N.V., 5656 AG Eindhoven (NL)
(72) Inventor: PALERO, Jonathan Alambra, Eindhoven (NL); DAMODARAN, Mathivanan, Eindhoven (NL)
(74) Representative: Philips Intellectual Property & Standards

(57) **Abstract**

A method for determining a parameter map for a region of a subject's body, the method comprising: obtaining (501), using polarised light incident on the region, at least three pairs of images of the region at respective different positions around the subject's body, wherein each pair comprises two images obtained at substantially the same position relative to the subject's body, wherein a first image in each pair is obtained using reflected light that has passed through a polariser with a first polarisation direction and a second image in each pair is obtained using reflected light that has passed through the polariser with a second polarisation direction, wherein the second polarisation direction is perpendicular or substantially perpendicular to the first polarisation direction; generating (503) one or more three-dimensional, 3D, reference meshes based on at least three images from at least three pairs of images; and determining (505) the parameter map based on the one or more 3D reference meshes and the at least three pairs of images.

## Description

### FIELD OF THE INVENTION

This disclosure relates to techniques for determining a parameter map for a region of a subject's body.

### BACKGROUND OF THE INVENTION

Skin measurement systems provide skin quantification and monitoring of features in the skin. Skin measurement systems can offer consumers information about features that are too small to detect with the naked eye, too faint to notice and/or are changing too slowly to follow.

For these systems to be acceptable to consumers, embedded sensing systems should be sensitive as well as specific. Robustness of measurement is essential to build consumer trust. Consumer engagement is achieved when reliable tracking and recognisable outcomes are achieved.

It is recognised herein that there exists a strong need to measure certain skin parameters and provide corresponding advice to the user. For example, measurement of the oiliness, glossiness, and/or radiance of skin is desired.

Gloss is a useful parameter to measure as it provides an indication of the effectiveness of skin care regimes. It is considered that 'good' skin should not be oily, but instead have a natural gloss. The gloss/oily appearance of the skin is the result of light interaction with the skin. The amount of gloss depends on the underlying surface and subsurface reflections arising from different angles of light incident on the skin. Gloss is important for skin beauty and has implications for the person's confidence.

Commercially available products that are capable of skin gloss measurements typically calculate gloss values from specular and diffuse light intensities.

Some gloss meters use two measurement channels to measure the direct (specular) and diffuse reflected light, and a diffuse scattering correction is applied to eliminate the portion of diffuse reflected light.

The gloss measurement device known as the SAMBA Face System consists of a high resolution digital camera equipped with a liquid-crystal polariser, the polarisation angle of which can be electronically flipped from the direction parallel (P) to the plane of polarisation of the polarising filters on the illumination units to the perpendicular direction, referred to as cross (C) orientation. The SAMBA device utilizes polarisation difference imaging and measures the polarisation state on each pixel in the entire region of interest and then calculates the average value of gloss based on the difference between the parallel and cross reflected components (P-C). This difference is the direct (specular) reflected light without the contribution from diffuse reflected light.

The SAMBA gloss measurement device, similar to other commercially available gloss meters, use device-dependent gloss units. The sensitivity of methods that are based on the ratio of specular to diffuse components of reflected light is lower in the low gloss regime (relevant for skin gloss characteristics) than in the high gloss regime.

Current techniques for measuring facial glossiness involve the following:
- The face is illuminated by a broadband polarised illumination. The light is reflected and scattered by the skin. The specular reflected light from the surface of the skin keeps the same polarisation as the incident illumination and the multiple scattering totally scrambles the light from deeper layers.
- A high frame-rate camera is used to capture the parallel and cross-polarised light at each spatial location of the face.
- Further image post-processing is performed using a simple subtraction or histogram analysis.

For example, the SAMBA Face system acquires successively two images of the parallel and cross-polarisation state (i.e. using parallel and cross parallel polarised light). These two images are then used to decompose the image obtained by a conventional camera (i.e. the image as seen by the eye) into two images: Specular and Diffuse. However, this device is very expensive and bulky.

Another way of measuring the gloss is using a skin gloss meter which is a stand-alone device employing diffuse scattering correction.

### SUMMARY OF THE INVENTION

In existing systems for measuring glossiness, the gloss measurements are either limited to a small region, or are evaluated over small regions of a wider area (e.g. a full face) where specular reflection is observed, e.g. as in the SAMBA Face system. In part, this is because the gloss measurements are obtained from a single position with respect to the region to be measured. However, it would be desirable to obtain and provide gloss measurements for a wider region, such as the full face. Therefore, there is a need for a technique that provides full-face mapping of skin glossiness.

According to a first aspect, there is provided a method for determining a parameter map for a region of a subject's body. The method comprises obtaining, using polarised light incident on the region, at least three pairs of images of the region at respective different positions around the subject's body, wherein each pair comprises two images obtained at substantially the same position relative to the subject's body, wherein a first image in each pair is obtained using reflected light that has passed through a polariser with a first polarisation direction and a second image in each pair is obtained using reflected light that has passed through the polariser with a second polarisation direction, wherein the second polarisation direction is perpendicular or substantially perpendicular to the first polarisation direction. The method further comprises generating one or more three-dimensional, 3D, reference meshes based on at least three images from at least three pairs of images; and determining the parameter map based on the one or more 3D reference meshes and the at least three pairs of images. This method provides mapping of various parameters across a distributed region of a subject's body. For example, the method provides full-face mapping of skin glossiness.

According to a second aspect, there is provided a computer program product comprising a computer readable medium having computer readable code embodied therein, the computer readable code being configured such that, on execution by a suitable computer or processing unit, the computer or processing unit is caused to perform the method according to the first aspect or any embodiment thereof.

According to a third aspect, there is provided an apparatus for determining a parameter map for a region of a subject's body. The apparatus comprises a processing unit configured to receive at least three pairs of images of the region obtained at respective different positions around the subject's body using polarised light incident on the region. Each pair comprises two images obtained at substantially the same position relative to the subject's body. A first image in each pair is obtained using reflected light that has passed through a polariser with a first polarisation direction and a second image in each pair is obtained using reflected light that has passed through the polariser with a second polarisation direction, wherein the second polarisation direction is perpendicular or substantially perpendicular to the first polarisation direction. The processing unit is further configured to generate one or more three-dimensional, 3D, reference meshes based on at least three images from at least three pairs of images and determine the parameter map based on the one or more 3D reference meshes and the at least three pairs of images.

According to a fourth aspect, there is provided a system comprising an apparatus according to the third aspect or any embodiment thereof. The system further comprises one or more of: a light source, the polariser, and an imaging unit.

Thus, the present invention provides a technique for mapping of skin glossiness of a region of a subject's body, such as the full face.

These and other aspects will be apparent from and elucidated with reference to the embodiment(s) described hereinafter.

### BRIEF DESCRIPTION OF THE DRAWINGS

Embodiments of the present disclosure will now be described, by way of example only, with reference to the following drawings, in which:
Fig. 1 is a block diagram of a system according to various embodiments;
Fig. 2 is a flow diagram illustrating a method according to various embodiments;
Fig. 3 illustrates different positions relative to a subject's head from which pairs of images can be obtained;
Fig. 4 is a flow diagram illustrating a method according to various embodiments;
Fig. 5 is a flow chart illustrating a method for determining a parameter map for a region of a subject's body; and
Fig. 6 is an illustration of a system according to various embodiments.

### DETAILED DESCRIPTION OF THE EMBODIMENTS

As noted above, techniques are required for providing full-face (or other body part) mapping of skin glossiness. Preferably the technique should be useable by a consumer, and so it should be possible for devices that implement the technique to be compact and/or portable. Therefore, the present disclosure provides a method and apparatus for determining a parameter map for a region of a subject's body. Herein, a parameter map is the name given to a structured representation of a plurality of parameter values for different locations across the region. The parameter values will usually be represented on a three-dimensional (3D) representation of the region, e.g. a 3D reconstruction, 3D render or polygon mesh. A parameter map therefore maps out the parameter values and illustrates variations of the parameter values across the region. This is achieved using multiple images of the region obtained from different positions with respect to the region.

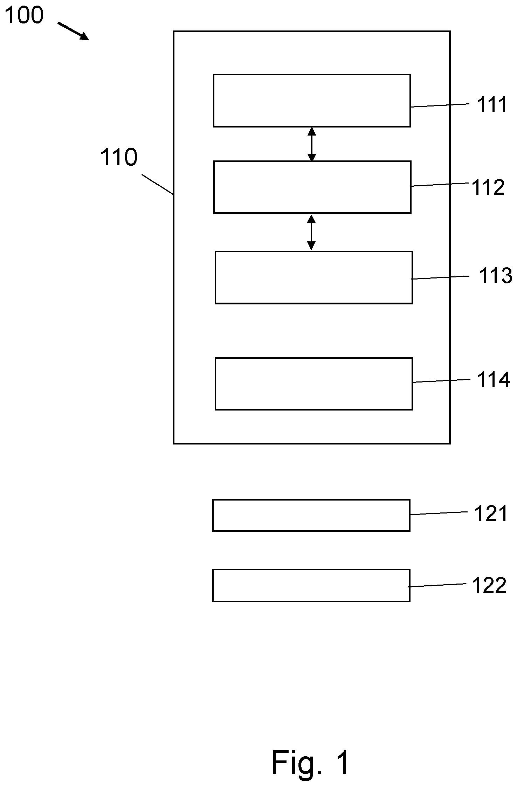

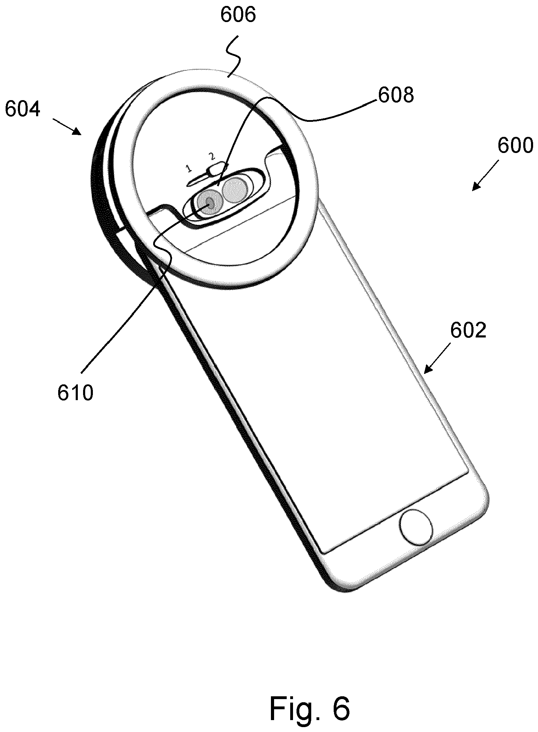

Fig. 1 is a block diagram of a system 100 for obtaining a parameter map according to some embodiments, the system 100 comprising an apparatus 110, a light source 121 and a polariser 122. In Fig. 1, the apparatus 110 is separate to the light source 121 and polariser 122. The apparatus 110 can be in the form of an electronic device, such as a smart phone (e.g. the mobile phone 602 of Fig. 6 below), smart watch, tablet, personal digital assistant (PDA), laptop, desktop computer, remote server, smart mirror, etc. In other embodiments, the light source 121 and/or polariser 122 can be part of, or integrated with, the apparatus 110.

In Fig. 1, there is an imaging unit 114 (e.g. camera) comprised in the apparatus 110. However, the imaging unit 114 may alternatively be integrated with the light source 121 and/or polariser 122, or it may be a separate unit to all of the apparatus 110, light source 121 and polariser 122.

The apparatus 110 in Fig. 1 also comprises a memory unit 111, a processing unit 112, and interface circuitry 113. It will be appreciated that in practice the apparatus 110 includes further components to those shown, for example a power source, a control unit, etc.

The imaging unit 114 is for obtaining a plurality of images. The imaging unit 114 may include any suitable components for capturing an image, for example a charge-coupled device (CCD) and one or more lenses and/or mirrors. In some embodiments, the imaging unit 114 is a camera, such as a digital camera. The polariser 122 is placed in front of the imaging unit 114 so that images can be obtained using light that has passed through the polariser 122.

The processing unit 112 generally controls the operation of the apparatus 110 and enables the apparatus 110 to perform the method and techniques described herein. Briefly, the processing unit 112 receives a plurality of images from the imaging unit 114 and processes the image(s) to determine a parameter map for a region of a subject's body. The processing unit 112 can also control the imaging unit 114 to obtain the plurality of images, and/or send instructions to be displayed to the user for obtaining the required images.

The processing unit 112 can be configured to receive the image(s) from the imaging unit 114, either directly in embodiments where the imaging unit 114 is part of the apparatus 110, or via another component in embodiments where the imaging unit 114 is separate from the apparatus 110. In either case, the processing unit 112 can include or comprise one or more input ports or wires for receiving the images (or signals carrying information representing the image(s)) from the imaging unit 114 or the other component as appropriate. The processing unit 112 can also include or comprise one or more output ports or wires for outputting a signal comprising the instructions and/or the parameter map. For example, the instruction and/or the parameter map may be displayed on a display that is part of the apparatus 110 or separate to the apparatus 110.

The processing unit 112 can be implemented in numerous ways, with software and/or hardware, to perform the various functions described herein. The processing unit 112 may comprise one or more microprocessors or digital signal processors (DSPs) that may be programmed using software or computer program code to perform the required functions and/or to control components of the processing unit 112 to effect the required functions. The processing unit 112 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor).

The processing unit 112 can comprise or be associated with a memory unit 111. The memory unit 111 can store data, information and/or signals (including image(s)) for use by the processing unit 112 in controlling the operation of the apparatus 110 and/or in executing or performing the methods described herein. In some implementations the memory unit 111 stores computer-readable code that can be executed by the processing unit 112 so that the processing unit 112 performs one or more functions, including the methods described herein. In particular embodiments, the program code can be in the form of an application for a smart phone, tablet, laptop, computer or server. The memory unit 111 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

The apparatus 110 also includes interface circuitry 113. The interface circuitry 113 in the apparatus 110 enables a data connection to and/or data exchange with other devices including any one or more of servers, databases, user devices, and sensors. For example, the interface circuitry 113 may provide a connection to a display unit, and/or light source 121 and polariser 122 when they are separate from the apparatus 110. In the embodiment shown in Fig. 1, the imaging unit 114 is included in the apparatus 110. However, as noted above, in some embodiments the imaging unit 114 may be separate from the apparatus 110, and in these embodiments the interface circuitry 113 enables the apparatus 110 to receive the image(s) from the imaging unit 114. Thus, the interface circuitry 113 in the apparatus 110 can enable a data connection to and/or data exchange with the imaging unit 114 (which may, for example, be comprised in the mapping device 120). The connection to the imaging unit 114, display unit, light source 121, polariser 122 or any other electronic device may be direct or indirect (e.g. via the Internet), and thus the interface circuitry 113 can enable a connection between the apparatus 110 and a network, or directly between the apparatus 110 and another device (such as imaging unit 114), via any desirable wired or wireless communication protocol. For example, the interface circuitry 113 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 113 (and thus apparatus 110) may include one or more suitable antennas for transmitting/receiving over a transmission medium (e.g. the air). Alternatively, in the case of a wireless connection, the interface circuitry 113 may include means (e.g. a connector or plug) to enable the interface circuitry 113 to be connected to one or more suitable antennas external to the apparatus 110 for transmitting/receiving over a transmission medium (e.g. the air). The interface circuitry 113 is connected to the processing unit 112.

Although not shown in Fig. 1, the apparatus 110 may comprise one or more user interface components that includes one or more components that enables a user of apparatus 110 to input information, data and/or commands into the apparatus 110, and/or enables the apparatus 110 to output information or data to the user of the apparatus 110. The user interface can comprise any suitable input component(s), including but not limited to a keyboard, keypad, one or more buttons, switches or dials, a mouse, a track pad, a touchscreen, a stylus, a camera, a microphone, etc., and the user interface can comprise any suitable output component(s), including but not limited to a display unit or display screen, one or more lights or light elements, one or more loudspeakers, a vibrating element, etc.

It will be appreciated that a practical implementation of an apparatus 110 may include additional components to those shown in Fig. 1. For example the apparatus 110 may also include a power supply, such as a battery, or components for enabling the apparatus 110 to be connected to a mains power supply.

The light source 121 is used to generate polarised light. For example, the polarised light may be generated to illuminate an 'imaging area' when the imaging unit 114 is to obtain images. According to the disclosure herein, the imaging area can be a region of a subject's body for which a parameter map is to be determined. The light source 121 may be a lamp with a polariser (this is a different polariser to the polariser 122 shown in Fig. 1). For example, the light source 121 may be one or more light emitting diodes (LEDs), with a suitable polarising component for polarising the light generated by the LEDs.

The polariser 122 shown in Fig. 1 is placed in front of the imaging unit 114 for polarising the light that is incident on the imaging unit 114. This light is sometimes referred to herein as reflected light. The plane of the polariser 122 may typically be perpendicular to the optical axis (i.e. principal axis) of the imaging unit 114. The polariser 122 polarises the light that passes through it according to a transmission axis (polarisation direction) of the polariser 122, and the imaging unit 114 captures an image using the polarised light. The polariser 122 can be rotated around the optical axis of the imaging unit 114 to change the polarisation direction with respect to the polarisation of the light generated by the light source 121. For example, the polarisation direction of the polariser 122 can be parallel to the polarisation direction of the light source 121 when some images are obtained by the imaging unit 114 and the polarisation direction of the polariser 122 can be perpendicular to the polarisation direction of the light source 121 when other images are obtained by the imaging unit 114. The polariser 122 may be, for example, an absorptive polariser or a wire grid polariser. Wire grid polarisers typically have a broader transmission spectrum (i.e. transmit more wavelengths) than absorptive polarisers. As noted above, in some embodiments, the polariser 122 may be comprised in the apparatus 110 or it may be separate to both the apparatus 110 and the light source 121.

Briefly, the techniques described herein provide for a parameter map to be determined for a region of a subject's body. Firstly, when polarised light from the light source 121 is incident on the region of interest, at least three pairs of images of the region are obtained at respective different positions around the subject's body using the imaging unit 114. Preferably the two images in each pair are obtained at substantially the same position relative to the subject's body, with one of the images in each pair being obtained when the polarisation direction of the polariser 122 is in a first direction, and the other image in each pair is obtained when the polarisation direction of the polariser 122 is in a second direction that is perpendicular or substantially perpendicular to the first polarisation direction. Next, one or more 3D reference meshes are generated based on at least three of the obtained images from at least three different pairs. Then, the parameter map is determined based on the one or more 3D reference meshes and the at least three pairs of images.

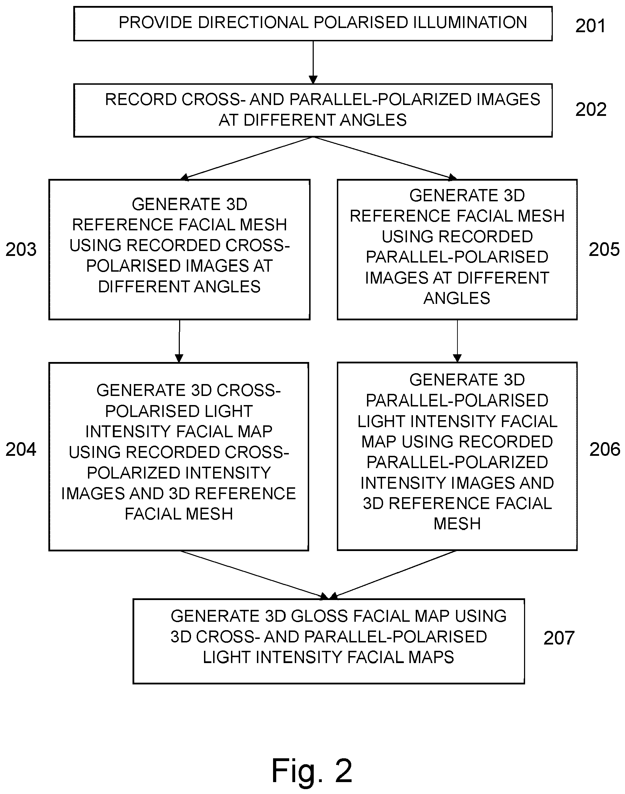

Fig. 2 is a flow chart illustrating a method for obtaining a skin glossiness facial map according to some embodiments of the invention. The method could be performed, for example, using the system 100 described with reference to Fig. 1.

At step 201, directional polarised illumination (i.e. light) is provided. The polarised light could be provided, for example, by the light source 121 of Fig. 1. The polarised light from the light source is referred to as being polarised in a "first" polarisation direction. The light is incident on a region of a subject's body for which a parameter map is to be obtained. For the discussion of Fig. 2, the region of the subject's body is the face, and the parameter is skin glossiness, although it will be appreciated that the region can be a different body part, such as an arm, leg, torso, etc., and/or the parameter can be a different parameter relating to the skin or region.

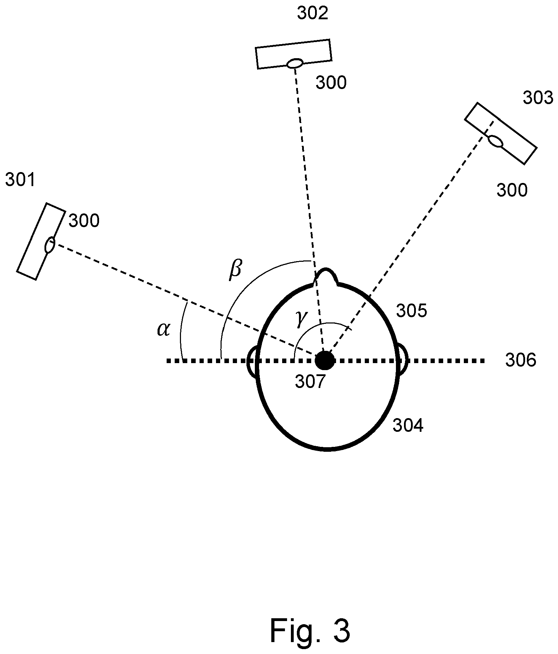

At step 202, cross- and parallel-polarised images are recorded from different angles relative to the region of the subject's body. These images could be obtained using an imaging unit, e.g. the imaging unit 114 described with reference to Fig. 1. To obtain the images from different angles, the imaging unit is placed at different positions around the subject's face. In some embodiments, each position is roughly the same distance from the face. These positions are described in more detail below with reference to Fig. 3.

To obtain cross-polarised images, the polariser in front of the imaging unit is orientated such that the polarisation direction of the polariser is perpendicular to the polarisation direction of the light emitted by the light source. To obtain parallel-polarised images, the polariser in front of the imaging unit is orientated such that the polarisation direction of the polariser is parallel to the polarisation direction of the light emitted by the light source. In some embodiments, images are obtained at at least three different angles relative to the subject's face, and at least one cross-polarised image and at least one parallel-polarised image is obtained for each angle.

The images can be taken in any order. For example, all of the cross-polarised images may be obtained first followed by all of the parallel-polarised images. Alternatively, all of the images from one angle may be obtained first (both parallel- and cross-polarised images), and the remaining images may be taken at each angle sequentially. Furthermore, the parallel-polarised images may be obtained first and the cross-polarised images second, or the cross-polarised images may be obtained first and the parallel-polarised images second.

The cross- and parallel-polarised images obtained from a particular angle/position are referred to as a pair of images, and thus in step 202 multiple pairs of images are obtained.

Fig. 3 illustrates different positions relative to a subject's head from which pairs of images can be obtained. Fig. 3 shows a top-down view of the subject 304 and a device 300 in three different imaging positions, 301, 302 and 303, with respect to the subject 304. In this embodiment, the images are of the subject's face 305.

The device 300 includes at least a light source (e.g. light source 121), an imaging unit (e.g. imaging unit 114) and a polariser (e.g. polariser 122) arranged in front of the imaging unit. The device 300 may include other components of the apparatus 110 or system 100 of Fig. 1. The polarised light emitted by the light source is incident on the face 305 of the subject 304.

The device 300 can be placed at position 301 and at least one parallel-polarised image is obtained when the polarisation axis/direction of the polariser is parallel to the polarisation direction of the light that is emitted by the light source and is incident on the subject's face 305.

At least one cross-polarised image can be obtained at position 301 when the polarisation axis/direction of the polariser is perpendicular to the polarisation direction of the light that is emitted by the light source and is incident on the subject's face 305.

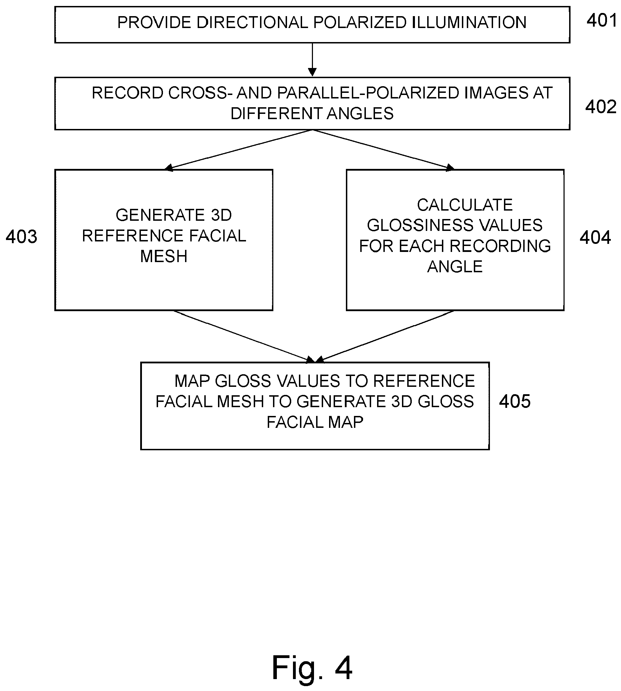

In some embodiments, the change in polarisation direction of the polariser can be executed manually by the user. In this case, the user may need to move the device 300 away from position 301 whilst changing the polarisation direction, e.g. by manually adjusting the position or orientation of the polariser in the device 300 relative to the imaging unit. The user will therefore move the device 300 approximately back to position 301 and obtain the image(s) using the oppositely-polarised light. Ideally, the device 300 is placed at exactly the same position 301, but the techniques described herein can still be effective if the device 300 is placed close to, but not necessarily at the exact same position 301 relative to the face (or other body region) for the cross- and parallel-polarised images from a given position/angle. In some embodiments, substantially the same position can mean within a few centimetres. Preferably, 'substantially the same position' means within 6 cm. For a camera held at arms-length and directed at the face of the person holding the camera (i.e. positioned for taking a selfie), a change in camera position of 6 cm results in a change in the position of the face in the image of approximately 10 mm. If this change in position of the face for a pair of images becomes too large, it will be more difficult to obtain the desired parameter values for the parameter map from the pair of images (e.g. as described with reference to step 207 of Fig. 2 and step 404 of Fig. 4).

In some embodiments, an application (e.g. on a smart phone) may guide the user to position the device 300 in substantially the same position relative to the subject's face 305 as it was for a previously obtained cross- or parallel-polarised image. The app may display directions (e.g. up, down, left, right etc.) on a screen, and/or a translucent copy of the previously obtained image may be displayed on the screen over the top of the current image being obtained so that the user can align the current image with the previously obtained image by moving and/or tilting the camera.

Pairs of cross- and parallel-polarised images are also obtained at positions 302 and 303 (as described for position 301).

The techniques disclosed herein make use of pairs of images obtained from at least three different positions relative to the region of the subject 304 to be mapped, and these positions can be defined by respective angles measured relative to an axis through the region. In Fig. 3 the three positions 301, 302 and 303 are defined by respective angles *α*, *β*, and *γ* measured around a longitudinal axis of the subject 304 and with respect to a frontal plane of the subject 304. Fig. 3 shows a dotted line 306 representing the frontal plane of the subject 304 and the angles are measured with respect to this plane 306 around a longitudinal axis 307 of the subject. However, for the purposes of this example, the angles could be measured from any plane through the region.

Position 301 is at an angle *α* to the plane 306, position 302 is at an angle *β* to the plane 306 and position 303 is at an angle *γ* to the plane 306. In some embodiments, the method requires that the range of these angles, i.e. the angle between extrema positions, is at most 180 degrees. In other words, the largest angle minus the smallest angle must be less than or equal to 180 degrees. In the example depicted in Fig. 3, this requirement can be expressed as |*γ* - *α*| ≤ 180°. This requirement ensures that the region of interest (e.g. the facial region) is fully captured by the images, i.e. that features of the region are not missed.

In some embodiments, the range of these angles must be at least 60 degrees. In other words, the largest angle minus the smallest angle must be at least 60 degrees. For example, this requirement can be used to ensure that the full face can be mapped, as described in "3D face reconstruction from mugshots: Application to arbitrary view face recognition" by Jie Liang et al., Neurocomputing 410 (2020) 12-27. In Fig. 3, this requirement can be expressed as |*γ* - *α*| ≥ 60°. In some embodiments, both this requirement and the previous requirement are applicable (i.e. 60 ≤ |*γ* - *α*| ≤ 180°).

In some embodiments, step 202 can further comprise performing ambient light correction on the obtained images. That is, light from the environment may be incident on the region of the subject 304 in addition to the polarised light from the light source, and this background/ambient light can prevent reliable parameter measurements being obtained in later steps of the method. The ambient (i.e. background) light contribution may vary across a single image and/or may vary between images due to the changing position of the imaging unit with respect to the face (i.e. as a function of the imaging angle). Therefore, the images may be processed to remove the contribution from ambient light. In these embodiments, the subsequent steps 203 to 207 are performed using the ambient-corrected images. Further information on ambient light correction can be found, for example, in WO 2021/089422 A1; WO 2021/122582 A1; EP 3869781 A1.

At step 203, a first 3D reference facial mesh is generated using the cross-polarised images obtained in step 202. These cross-polarised images of the region were obtained from different angles with respect to the face 305. Therefore, the first 3D reference facial mesh combines details of the face 305 across a wider range of views than can be obtained from a single image.

The first 3D reference facial mesh may be generated by fitting specific feature pixels obtained from the cross-polarised images into a generic 3D facial model that can be morphed based on the location (i.e. position) data of the feature pixels.

The specific feature pixels can be selected from facial features such as eyebrow boundaries, eye corners, nose contours, nose tip, mouth boundaries and face contours. In some embodiments, these feature pixels can be manually annotated. Alternatively, the feature pixels can be automatically extracted using an appropriate detection algorithm.

The generic 3D morphable facial model can be derived by learning a database of facial scans as a vector space representation. For example, "Face recognition based on fitting a 3D morphable model" by V. Blanz and T. Vetter, published in IEEE Transactions on Pattern Analysis and Machine Intelligence, Volume 25, Issue 9, 2003, derived a 3D morphable model (3DMM) based on a set of 200 3D facial scans.

At step 204, cross-polarised light intensities are mapped onto the first 3D reference facial mesh generated in step 203 to generate a 3D cross-polarised light intensity facial map, also referred to herein as the first 3D light intensity map. The cross-polarised light intensities are the pixel intensities in the 2D cross-polarised images recorded in step 202. The cross-polarised light intensities correspond to light that has been reflected from the region with a polarisation that is perpendicular to the direction of polarisation of the light that was incident on the region, and thus the cross-polarised light includes contributions from diffuse (unpolarised) reflections from the region.

The mapping of the cross-polarised light intensities onto the first 3D reference facial mesh can be performed as follows. For each cross-polarised image, the 3D location of each image pixel on the region of interest (e.g. the face) is derived from the fitted 3D model obtained in step 203. More specifically, the 3D location of each image pixel in the cross-polarised image can be derived from the fitted 3D location (i.e. position) coordinates of the feature pixels in the cross-polarised image. The light intensities for each image pixel in the cross-polarised images can then be mapped onto the first 3D reference facial mesh using the obtained 3D location data for the image pixels. Further details about determining a 3D reference facial mesh can be found, for example, in "Three-Dimensional Face Reconstruction Using Multi-View-Based Bilinear Model" by L. Tian et al., Sensors - Multidisciplinary Digital Publishing Institute, 4 December 2018.

The light intensity, also referred to as image intensity or pixel intensity, is independent of colour. This colour-independent intensity or lightness can be obtained in a number of ways: (1) using a Red Green Blue (RGB) colour camera and selecting one colour channel, e.g., Green channel; (2) selecting two or more colour channels and summing them up, e.g. R+G+B; or (3) using a single-colour or monochrome camera.

Steps 205 and 206 are analogous to steps 203 and 204 but are performed for the parallel-polarised images in the image pairs instead of the cross-polarised images. Steps 205 and 206 can be performed before, after, or at the same time as steps 203 and 204.

Thus, at step 205, a second 3D reference facial mesh is generated using the parallel-polarised images obtained in step 202. As noted above, these images are obtained from different angles with respect to the face. Step 205 can be performed in a similar way to step 203 above.

At step 206, parallel-polarised light intensities are mapped onto the second 3D reference facial mesh generated in step 205 to generate a 3D parallel-polarised light intensity facial map, also referred to herein as the second 3D light intensity map. The parallel-polarised light intensities are the pixel intensities in the 2D parallel-polarised images recorded in step 202. Parallel-polarised light intensities correspond to light that has been reflected from the region with a polarisation direction that is parallel to the polarisation direction of the light that was incident on the region. This light will include both specular reflections and diffuse (unpolarised) reflections from the region. Step 206 can be performed in a similar way to step 204 above.

Skin gloss (also referred to as glossiness) at any given location on the skin can be calculated as the ratio of specular reflectance to diffuse reflectance at that location. The specular reflected light intensity at any given location of the face (or any other region of the subject) can be determined based on the light intensity obtained by subtracting the cross-polarised light intensity for the particular location on the face/region from the parallel-polarised light intensity for that location. This is because the parallel-polarised light intensity includes a contribution from diffuse reflections, and this contribution can be removed by subtracting the cross-polarised light intensity. The diffuse reflected light intensity at a given location is equal to the cross-polarised light intensity at that location. Thus, the skin gloss/glossiness is obtained using the ratio of the specular reflected light intensity to the diffuse reflected light intensity. This can be expressed as Gloss = Specular/Diffuse and therefore Gloss = (I_Parallel - I_Cross)/I_Cross, where I represents intensity of reflected light.

According to the method illustrated in Fig. 2, in step 207 a 3D skin gloss facial map is generated based on the 3D cross- and parallel-polarised light intensity facial maps generated in steps 204 and 206. The 3D cross-polarised intensity map determined in step 204 provides respective intensity values for a large number of locations on the face. Likewise the 3D parallel-polarised intensity map determined in step 206 provides intensity values for those same locations on the face. Thus, it is possible to subtract the cross-polarised light intensity values from the parallel-polarised light intensity values, and divide the result by the cross-polarised light intensities to determine skin glossiness values at those facial locations. These skin glossiness values are then presented as a 3D gloss facial map.

Fig. 4 is a flow chart illustrating a method for obtaining a skin glossiness facial map according to some alternative embodiments of the invention. The method could be performed using the system 100 described with reference to Fig. 1.

At step 401, directional polarised illumination is provided. Step 401 can be performed in the same way as step 201 described above.

At step 402, cross- and parallel-polarised images are recorded at different angles relative to the subject's face. Step 402 can be performed in the same way as step 202 described above. For example, a pair of images can be obtained at each angle, where one image in each pair is obtained using cross-polarised reflected light (i.e. the polarisation direction of the polariser is perpendicular to the polarisation direction of the light emitted by the light source) and the other image in each pair is obtained using parallel-polarised reflected light (i.e. the polarisation direction of the polariser is parallel to the polarisation direction of the light emitted by the light source).

In some embodiments, step 402 can further comprise performing ambient light correction on the obtained images, for example as described above with respect to step 202.

At step 403, a 3D reference facial mesh is generated using a plurality of the images obtained in step 402. The plurality of images include images obtained from different angles with respect to the face. Therefore, the 3D reference facial mesh combines details of the face across a wider range of views than can be obtained from a single imaging position.

In some embodiments, the 3D reference facial mesh is generated using only the parallel-polarised images obtained in step 402. This is advantageous because parallel-polarised reflected light shows more features, e.g. due to specular reflection from pores, lips, hair, etc. These features can be useful for selecting the feature pixels. In alternative embodiments, the 3D reference facial mesh is generated using some or all of the cross-polarised and parallel-polarised images obtained in step 402.

The 3D reference facial mesh can be generated using the method described with reference to steps 203 and 205 of Fig. 2. That is to say, specific feature pixels can be obtained from the images obtained in step 402 and fitted into a generic 3D facial model. The 3D facial model can be morphed based on the location data of the feature pixels.

At step 404, for each recording angle (i.e. each pair of images), gloss values are calculated for different parts of the region (i.e. for different locations on the region) using the images obtained in step 402. For example, the light intensity obtained for a particular location in the cross-polarised image at a given recording angle can be subtracted from the light intensity obtained for the particular location in the parallel-polarised image at the same recording angle. Dividing this value by the cross-polarised light intensity for the location provides a gloss value for that location. The result is a set of gloss/glossiness values for different locations on the region for each pair of images taken from a given recording angle. Step 404 can be performed before, after or at the same time as step 403.

In step 405, the gloss/glossiness values obtained in step 404 are mapped onto the 3D reference facial mesh obtained in step 403 to generate a 3D gloss facial map. The mapping of gloss/glossiness values onto the 3D reference facial map in this step can be performed using similar techniques to those employed in step 204 of Fig. 2 for mapping light intensities onto a 3D reference facial map. However, instead of using the pixel intensities in the images (as in step 204), in step 405 the gloss values (which can be visualised by grey scale values) are used. Firstly, each of the gloss values obtained in step 404 are associated with 3D location data. The 3D location on the face of each gloss value can be derived from the fitted 3D model obtained in step 403. More specifically, the 3D location of a given gloss value obtained from an image pair can be derived from the fitted 3D location coordinates of the feature pixels that were identified in that image pair. Using this 3D location data for each gloss value, the gloss values can be mapped onto the 3D reference facial mesh.

In some cases, different gloss values are obtained for the same location on the face because different image pairs captured from different angles give rise to different gloss values. In such cases, there are various possible methods for obtaining a single gloss value for that location to be included on the 3D gloss facial map. In some embodiments, a single gloss value can be obtained by determining a simple average of the obtained gloss values for that location on the region. In alternative embodiments, the maximum gloss value obtained for that location on the region may be used. In further embodiments, a weighted average of the obtained gloss values may be determined. The weighted average may be based on the relationship between the optical axis (i.e. principal axis) of the imaging unit and the normal to the skin surface (i.e. the direction perpendicular to the skin surface), such that gloss values obtained with the optical axis of the imaging unit closer to the skin surface normal are weighted more strongly than those obtained with the optical axis of the imaging unit further from the normal. This is because gloss values calculated for a skin surface that is orthogonal to the optical axis are in general more accurate, and deviation from the normal decreases the accuracy of the obtained gloss value.

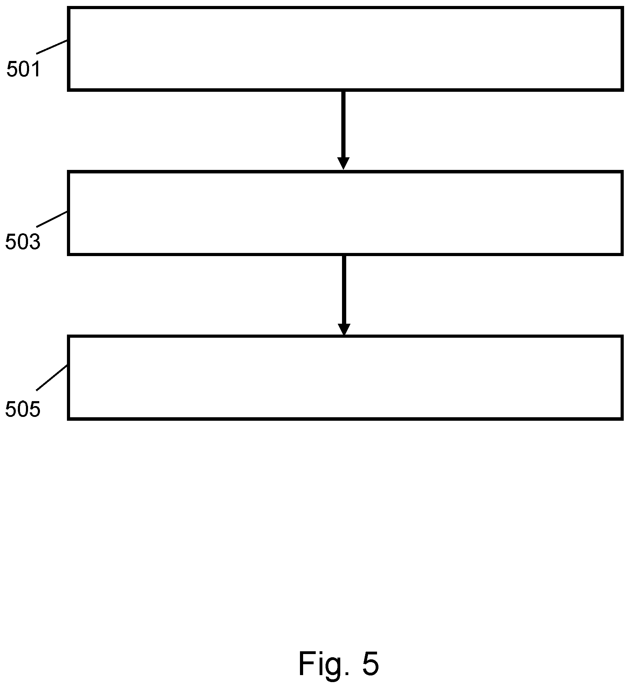

Fig. 5 is a flow chart illustrating a method for determining a parameter map for a region of a subject's body according to the techniques described herein. In some embodiments, the parameter map comprises a representation of a plurality of parameter values for different locations on the region. The region could be any region of the subject's body, for example the face, scalp, hand, foot, back, chest or leg. The parameter could be any parameter that is related to, or can be determined based on, specular reflectance and/or diffuse reflectance. For example, the parameter could be skin gloss/glossiness, skin tone, specular light intensity, hair gloss/glossiness, hair colour, hair count or facial structure. The method of Fig. 5 could be carried out using the system described with reference to Fig. 1 or Fig. 6.

The method comprises, at step 501 of Fig. 5, obtaining, using polarised light incident on the region, at least three pairs of images of the region at respective different positions around the subject's body. The source of the polarised light could be the light source 121 of Fig. 1 or the polarised LED ring 606 that is described with reference to Fig. 6. The polarised light could correspond to the light provided in step 201 of Fig. 2 or step 401 of Fig. 4.

The at least three pairs of images may be obtained using an imaging unit and a polariser. The imaging unit could be, for example, the imaging unit 114 of Fig. 1, an imaging unit incorporated into the device 300 of Fig. 3, or the camera 610 of Fig. 6. The polariser may be, for example, the polariser 122 of Fig. 1 or the sliding/switchable dual-polariser 608 described with reference to Fig. 6. The polariser may be positioned between the region of the subject's body and the imaging unit.

Obtaining the at least three pairs of images of the region at different positions around the subject's body in step 501 may comprise obtaining the at least three pairs of images with the imaging unit at different positions with respect to the region. The positions could be, for example, the imaging positions 301, 302 and 303, described with reference to Fig. 3. Obtaining the at least three pairs of images of the region could, for example, correspond to step 202 of Fig. 2 or step 402 of Fig. 4.

In some embodiments, the method may further comprise, prior to obtaining the at least three pairs of images of the region, determining a suitable distance of the imaging unit to the region, and guiding a user to move the imaging unit to the determined distance. The distance may be determined based on one or more of: the size of the region, the shape of the region, the image processing method to be used, and the imaging unit. The guidance could be in the form of visual instructions displayed on a screen (e.g. the display unit or display screen described with reference to Fig. 1), audio instructions played via a speaker or other audio device (e.g. headphones), or other sensory feedback, e.g. by vibrating a user device (e.g. device 300 or apparatus 110) when the user has moved the imaging unit to the determined distance from the region. The skilled person will be aware of alternative methods for guiding a user to move the imaging unit to the determined distance.

Each image pair comprises two images obtained at substantially the same position relative to the subject's body. In some embodiments, substantially the same position can mean within a few centimetres in any direction, e.g., within 6 cm. Further discussion of the meaning of 'substantially the same position' is provided with reference to Fig. 3.

A first image in each pair is obtained using reflected light that has passed through the polariser in a first polarisation direction. Having the polariser 'in the first polarisation direction' means that the polariser is configured (e.g. orientated) such that only light that is polarised in the first direction is transmitted by the polariser. The reflected light is the light that is incident on the imaging unit and includes light that has been reflected and/or scattered by the region. The light that is incident on the imaging unit may also include ambient light.

A second image in each pair is obtained using reflected light that has passed through the polariser with a second polarisation direction. The polarisation direction of the polariser is therefore changed between obtaining the first image and the second image in any given image pair.

In some embodiments, the method further comprises, after obtaining a first image of an image pair with the imaging unit in a first position relative to the subject's body, guiding a user to move the imaging unit to substantially the first position relative to the subject's body for obtaining a second image in the image pair. In some embodiments, an application (e.g. on a smart phone) may perform the guiding step. The application may display directions (e.g. up, down, left, right etc. or graphical representations thereof) on a screen, and/or a translucent copy of the first image in the pair may be displayed on the screen over the top of (e.g. superimposed on) the current image being obtained so that the user can align the current image with the previously obtained image by moving and/or tilting the imaging unit.

The second polarisation direction is perpendicular or substantially perpendicular to the first polarisation direction. The polarisation direction of the reflected light may be described with respect to the polarisation direction of the polarised light that is incident on the region. In some embodiments, the first polarisation direction is perpendicular (or substantially perpendicular) to a polarisation direction of the polarised light incident on the region, and the second polarisation direction is parallel (or substantially parallel) to the polarisation direction of the polarised light incident on the region. Substantially perpendicular/parallel may mean within 10 degrees of being perpendicular/parallel.

The method further comprises generating, at step 503, one or more 3D reference meshes based on at least three images from at least three pairs of images, and determining, at step 505, the parameter map based on the one or more 3D reference meshes and the at least three pairs of images.

In some embodiments, the parameter is skin gloss/glossiness and the skin gloss/glossiness map is determined based on a difference between reflected light intensity for images obtained with the polariser in the first polarisation direction and reflected light intensity for images obtained with the polariser in the second polarisation direction.

In some embodiments, the method further comprises, prior to determining the parameter map in step 505, performing ambient light correction on the obtained images. This step improves the determined parameter map by providing more accurate images of the region. Performing ambient light correction on the obtained images can also increase the detectability of features in the images.

In some embodiments, generating one or more 3D reference meshes comprises generating a first 3D reference mesh based on the first images in each pair and generating a second 3D reference mesh based on the second images in each pair. Generating the first 3D reference mesh may correspond to step 203 of Fig. 2. In these embodiments, the first 3D reference mesh may correspond to the 3D reference facial mesh generated using cross-polarised images at different angles. Generating the second 3D reference mesh may correspond to step 205 of Fig. 2. In these embodiments, the second 3D reference mesh may correspond to the 3D reference facial mesh obtained using recorded parallel-polarised images at different angles.

In these embodiments, step 505 of determining the parameter map may comprise generating a first 3D light intensity map for the region based on the first 3D reference mesh and the first images. Generating the first 3D light intensity map may comprise mapping light intensities from the first images onto the first 3D reference mesh. Generating the first 3D light intensity map may correspond to step 204 of Fig. 2.

Step 505 of determining the parameter map may further comprise generating a second 3D light intensity map for the region based on the second 3D reference mesh and the second images. Generating the second 3D light intensity map may comprise mapping light intensities from the second images onto the second 3D reference mesh. Generating the second 3D light intensity map may correspond to step 206 of Fig. 2.

Step 505 may further comprise a step of determining the parameter map based on the first and second 3D light intensity maps. This step may correspond to step 207 of Fig. 2. The parameter map may provide parameter values for a plurality of locations on the region. The parameter map may illustrate these values on a 3D map/mesh.

In alternative embodiments, step 503 of generating one or more 3D reference meshes comprises generating a third 3D reference mesh. In these embodiments, the third 3D reference mesh may be the only 3D reference mesh generated based on the pairs of images obtained in step 501. Generating the third 3D reference mesh may correspond to step 403 of Fig. 4.

In these embodiments, determining the parameter map in step 505 may comprise determining a plurality of parameter values for a corresponding plurality of locations on the region. Each parameter value is based on at least one of the obtained pairs of images. Determining the parameter value for a corresponding location may comprise determining, based on at least one of the obtained pairs of images, an intensity of light reflected from the location in the first image in the pair and an intensity of light reflected from the location in the second image in the pair, and determining the parameter value based on these determined intensities. For example, the gloss/glossiness at a given location can be obtained by subtracting the intensity of cross-polarised light reflected from that location from the intensity of parallel-polarised light reflected from that location and dividing the result by the intensity of cross-polarised reflected light from that location. This step of determining a plurality of parameter values may correspond to step 404 of Fig. 4.

In some of these embodiments, determining the parameter map in step 505 may further comprise mapping the parameter values onto corresponding locations on the third 3D reference mesh. This mapping step may correspond to step 405 of Fig. 4.

Fig. 6 is an illustration of a system for determining a parameter map for a region of a subject's body according to some embodiments. It will be appreciated that the system 600 in Fig. 6 is merely presented as an example of a system 600 with which the invention can be used, and the system 600 is not limited to the form shown in Fig. 6. In particular, the techniques disclosed herein can be realised with a mobile phone (e.g. smartphone), but the techniques are not limited to embodiments that make use of a mobile phone.

The system 600 comprises a mobile phone 602 (e.g. smartphone) equipped with a clip-on device 604. The clip-on device 604 comprises a polarised LED ring 606 and a sliding or switchable dual-polariser 608. The clip-on device 604 can be attached to and removed from a mobile phone 602. Fig. 1 shows the clip-on device clipped onto the mobile phone 602 so that the sliding/switchable dual polariser 608 is over a camera 610 of the mobile phone 602. The camera 610 may be the front (selfie) camera of the mobile phone 602 if the user of the phone 602 is obtaining a parameter map of themselves, or camera 610 may be the rear (main) camera of the mobile phone 602 if the user of the phone 602 is obtaining a parameter map of someone else.

The polarised LED ring 606 is configured to emit polarised light. The sliding dual-polariser 608 has two polarisers oriented perpendicular and parallel to the polarisation direction of the polarised light emitted from the polarised LED ring 606, with one of the two polarisers being selectively positioned over the front camera 610 of the mobile phone 602. The illumination and/or camera 610 can be controlled by an application (also referred to herein as an app) on the mobile phone. The app may guide the user during image capturing, process the captured images, and display the results to the user.

The clip-on device 604 may include attaching means (e.g. a clip, a suction cup, a hook and loop arrangement) for attaching the device 604 to the mobile phone 602, and for enabling the clip-on device 604 to be detached from the mobile phone 602.

In some embodiments, the system 600 may be configured to perform the following steps:
1. A user is guided by the app to configure the dual-polariser 608 so that the cross-polariser is in a position covering the front camera 610;
2. The user is guided to hold the smartphone 602 at an optimal distance from the subject's face;
3. The user enables the start of capturing of cross-polarised images of the face;
4. The user is guided to move the smartphone 602 to capture images from different angles of the face;
5. The app may automatically stop collecting images within a specified time, or after a specified number of images are captured;
6. The user is then guided to the next step to capture parallel-polarised images;
7. The user is guided by the app to configure the dual-polariser 608 so that the parallel-polariser is in a position covering the front camera 610;
8. The user is guided to hold the smartphone 602 at an optimal distance from the face;
9. The user enables the start of capturing of parallel-polarised images;
10. The user is guided to move the smartphone sideways to capture different angles of the face;
11. The app may automatically stop collecting images within a specified time, or after a specified number of images are captured;
12. The app processes the two sets of images (the pairs of cross- and parallel-polarised images at different angles) to produce a facial map of skin gloss/glossiness according to any of the methods in Figs. 2, 4 or 5. For example, the app may calculate the gloss values according to the method in Fig. 5 and map them onto one facial map of skin gloss/glossiness.

In this example, the parameter being measured is skin gloss/glossiness, and the region of the subject is the subject's face. However, as noted above, the techniques described herein are applicable to a wide range of parameters and body regions. For example, 3D gloss/glossiness maps can be obtained for any body region, such as the face, scalp, back, chest, hand or leg. The resulting 3D gloss maps could be used for various mapping applications including skin oiliness across any body region. The relative skin oiliness map may be combined with a pore map to provide insights on acne-prone regions of the face, and thus help mitigate acne breakouts by applying spatially selective treatment.

Skin glossiness is determined based on specular reflections from the skin, and therefore a skin glossiness map is based on a 3D map of specular reflections. 3D maps of specular reflections can also be used for, for example, highlighting skin structures such as wrinkles, fine lines and pores.

In other embodiments, the body region could be the head for scalp hair characterisation. For example, the techniques disclosed herein can be used to measure hair glossiness and generate a 3D hair glossiness map.

Therefore, there is provided a method, apparatus and system for determining a parameter map for a region of a subject's body.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method for determining a parameter map for a region of a subject's body, the method comprising:
obtaining (501), using polarised light incident on the region, at least three pairs of images of the region at respective different positions around the subject's body, wherein each pair comprises two images obtained at substantially the same position relative to the subject's body, wherein a first image in each pair is obtained using reflected light that has passed through a polariser with a first polarisation direction and a second image in each pair is obtained using reflected light that has passed through the polariser with a second polarisation direction, wherein the second polarisation direction is perpendicular or substantially perpendicular to the first polarisation direction;
generating (503) one or more three-dimensional, 3D, reference meshes based on at least three images from at least three pairs of images; and
determining (505) the parameter map based on the one or more 3D reference meshes and the at least three pairs of images.

2. A method as claimed in claim 1, wherein generating (503) one or more 3D reference meshes comprises:
generating a first 3D reference mesh based on the first images in each pair; and
generating a second 3D reference mesh based on the second images in each pair.

3. A method as claimed in claim 2, where determining (505) the parameter map comprises:
generating a first 3D light intensity map for the region based on the first 3D reference mesh and the first images;
generating a second 3D light intensity map for the region based on the second 3D reference mesh and the second images; and
determining the parameter map based on the first and second 3D light intensity maps.

4. A method as claimed in claim 3, wherein generating the first 3D light intensity map comprises mapping light intensities from the first images onto the first 3D reference mesh, and wherein generating the second 3D light intensity map comprises mapping light intensities from the second images onto the second 3D reference mesh.

5. A method as claimed in claim 1, wherein generating (503) one or more 3D reference meshes comprises generating a third 3D reference mesh, and wherein determining (505) the parameter map comprises:
determining a plurality of parameter values for a corresponding plurality of locations on the region, wherein each parameter value is based on at least one of the obtained pairs of images; and
mapping the parameter values onto corresponding locations on the third 3D reference mesh.

6. A method as claimed in claim 5, wherein determining the parameter value for a corresponding location comprises:
determining, based on at least one of the obtained pairs of images, an intensity of light reflected from the location in the first image in the pair and an intensity of light reflected from the location in the second image in the pair; and
determining the parameter value based on the determined intensities.

7. A method as claimed in any of claims 1-6, wherein the step of obtaining (501) is performed using an imaging unit and the polariser.

8. A method as claimed in any of claims 1-7, wherein the parameter is one of: skin gloss/glossiness, skin tone, specular light intensity, hair gloss/glossiness, hair colour, hair count and facial structure.

9. A method as claimed in any of claims 1-8, wherein the parameter map comprises a representation of a plurality of parameter values for different locations on the region.

10. A method as claimed in any of claims 1-9, wherein the parameter is skin gloss/glossiness, and the skin gloss/glossiness map is determined based on a difference between reflected light intensity for images obtained with the polariser in the first polarisation direction and reflected light intensity for images obtained with the polariser in the second polarisation direction.

11. A method as claimed in any of claims 1-10, wherein the first polarisation direction is perpendicular to a polarisation direction of the polarised light incident on the region, and the second polarisation direction is parallel to the polarisation direction of the polarised light incident on the region.

12. A computer program product comprising a computer readable medium having computer readable code embodied therein, the computer readable code being configured such that, on execution by a suitable computer or processing unit, the computer or processing unit is caused to perform the method of any of claims 1-11.

13. An apparatus (110) for determining a parameter map for a region of a subject's body, the apparatus (110) comprising a processing unit (112) configured to:
receive at least three pairs of images of the region obtained at respective different positions around the subject's body using polarised light incident on the region, wherein each pair comprises two images obtained at substantially the same position relative to the subject's body, wherein a first image in each pair is obtained using reflected light that has passed through a polariser with a first polarisation direction and a second image in each pair is obtained using reflected light that has passed through the polariser with a second polarisation direction, wherein the second polarisation direction is perpendicular or substantially perpendicular to the first polarisation direction;
generate one or more three-dimensional, 3D, reference meshes based on at least three images from at least three pairs of images; and
determine the parameter map based on the one or more 3D reference meshes and the at least three pairs of images.

14. An apparatus (110) as claimed in claim 13, the apparatus (110) further configured to perform the method of any of claims 2-12.

15. A system (100) comprising an apparatus (110) according to claim 13 or 14, the system (100) further comprising one or more of: a light source (121), the polariser (122), and an imaging unit (114).
